# DEMANDE DE BREVET EUROPEEN

(11) **EP 3 502 635 A1**
(43) Date de publication de la demande: **26.06.2019**
(21) Numéro de dépôt: 18212918.9
(22) Date de dépôt: 17.12.2018
(51) Int. Cl.: G01G 19/50

(54) **PROCÉDÉ DE COMMANDE D'UN DISPOSITIF ÉLECTRONIQUE DE PESÉE**

(30) Priorité: 21.12.2017 FR 1762758
(71) Demandeur: SEB S.A., 69130 Ecully (FR)
(72) Inventeur: GHECHOUA, Karim, 69800 SAINT PRIEST (FR)
(74) Mandataire: Soares, Luis Filipe

(57) **Abrégé**

Procédé de commande d'un dispositif électronique de pesée (3) comprenant au moins une première électrode (17) et une seconde électrode (17), le procédé comprenant une étape de mise en contact de la première électrode (17) avec un emplacement prédéterminé d'une partie droite du corps d'un utilisateur, et de la seconde électrode avec un emplacement prédéterminé d'une partie gauche du corps de l'utilisateur; une étape d'acquisition d'un signal électrocardiographique (21) entre la première et la seconde électrode (17, 19) ; une étape de sélection, à partir du signal électrocardiographique acquis (21), d'un identifiant unique (43) particulier ; une étape d'élaboration d'une information de commande (11), l'information de commande (11) étant élaborée en fonction du profil utilisateur (29) associé à l'identifiant unique (43) sélectionné.

## Description

### Domaine de l'invention

La présente invention concerne un procédé de commande d'un dispositif électronique de pesée.

### Art antérieur

Il est connu de définir un profil utilisateur comprenant des préférences d'utilisation d'un dispositif électronique de pesée. Cette disposition permet à l'utilisateur dont le profil est défini d'avoir accès à des commandes spécifiques qu'il utilise régulièrement.

L'utilisateur doit alors se connecter sur son profil qui est enregistré sur le dispositif électronique de pesée. Ensuite, ses commandes favorites peuvent être mises à sa disposition par une interface utilisateur comme par exemple un écran tactile.

Ce mode de fonctionnement donne satisfaction en ce que l'utilisateur trouve facilement ses commandes préférées. Cependant l'utilisateur doit tout d'abord se connecter sur son profil ce qui nécessite des interactions avec l'interface utilisateur de sa part.

Ainsi le temps gagné par la mise à disposition des commandes préférées peut être contrebalancé par le temps nécessaire à la connexion au profil utilisateur.

Ceci est particulièrement vrai pour un dispositif électronique de pesée utilisé par plusieurs personnes car un maintien de la connexion sur un profil utilisateur donné entre deux utilisations n'est d'aucune utilité.

La présente invention vise à résoudre tout ou partie des inconvénients mentionnés ci-dessus.

### Exposé de l'invention

A cet effet, la présente invention concerne un procédé de commande d'un dispositif électronique de pesée comprenant au moins une première électrode et une seconde électrode, le procédé comprenant :
une étape de mise en contact de la première électrode avec un emplacement prédéterminé d'une partie droite du corps d'un utilisateur, et de la seconde électrode avec un emplacement prédéterminé d'une partie gauche du corps de l'utilisateur, la partie droite et la partie gauche du corps étant entendues comme étant respectivement du côté droit et du côté gauche du plan parallèle au plan sagittal du corps de l'utilisateur et traversant le coeur ;
une étape d'acquisition d'un signal électrocardiographique entre la première et la seconde électrode ;
une étape de sélection, à partir du signal électrocardiographique acquis, d'un identifiant unique particulier parmi une pluralité d'identifiants uniques enregistrés dans une base de données, chaque identifiant unique de la base de données étant associé d'une part à un signal électrocardiographique de référence enregistré dans la base de données et d'autre part à un profil utilisateur enregistré dans la base de données, ledit profil utilisateur comprenant des préférences d'utilisation du dispositif électronique de pesée ;
une étape d'élaboration d'une information de commande, l'information de commande étant élaborée en fonction du profil utilisateur associé à l'identifiant unique sélectionné.

Cette disposition permet de simplifier la procédure d'élaboration de l'information de commande par l'utilisateur. En effet, le nombre d'interactions entre l'utilisateur et le dispositif électronique de pesée est limité puisque l'utilisateur n'a pas à entrer plusieurs commandes manuelles pour que l'information de commande soit élaborée.

Ainsi un utilisateur dont le profil est connu pourra commander de manière aisée le dispositif électronique de pesée puisque ses préférences d'utilisation sont connues.

Selon un aspect de l'invention, l'étape de sélection de l'identifiant unique particulier est établie selon une comparaison du signal électrocardiographique acquis avec les signaux électrocardiographiques de référence de la base de données.

Cette disposition permet de réaliser une identification simple, rapide et fiable dans le sens où la probabilité de confondre deux utilisateurs est limitée.

Selon un aspect de l'invention, l'information de commande correspond à une commande d'enregistrement d'une information de mesure provenant d'une unité de mesure du dispositif électronique de pesée.

Cette disposition permet d'utiliser une unité de mesure telle qu'un ruban mesureur d'une longueur ou une balance, l'information de mesure enregistrée étant liée au profil utilisateur sélectionné.

Selon un aspect de l'invention, l'information de commande correspond à une commande de mise à disposition par le dispositif électronique de pesée d'un élément d'interface utilisateur pourvu d'une sélection d'au moins une commande de fonctionnement parmi une pluralité de commandes de fonctionnement existantes du dispositif électronique de pesée.

Cette disposition permet de limiter les choix proposés à l'utilisateur selon ses préférences d'utilisation. Aucune commande de fonctionnement que l'utilisateur ne sélectionne rarement ou qu'il ne souhaite pas utiliser n'est proposée.

Selon un aspect de l'invention, les étapes décrites ci-avant sont répétées, l'emplacement prédéterminé de la partie droite et respectivement l'emplacement prédéterminé de la partie gauche de l'utilisateur étant identiques à chaque étape d'acquisition d'un signal électrocardiographique.

Pour faire de l'identification par électrocardiographie, l'emplacement des mesures sur le corps doit être identique à chaque acquisition.

Les première et seconde électrodes utilisées pour de telles mesures sont passives. Une différence de potentiel est mesurée entre la partie droite et la partie gauche du corps de l'utilisateur par rapport au coeur. Le terme électrode est à comprendre comme plot conducteur.

Dans le corps humain, les muscles sont commandés, pilotés, par le système nerveux qui fait transiter des impulsions électriques. Le coeur étant un muscle, il travaille sous le rythme d'impulsions électriques. L'électrocardiographie consiste à capter ces impulsions électriques.

Le signal électrocardiographique dépend du positionnement des emplacements prédéterminés du corps sur lesquels sont mises en contact les première et seconde électrodes.

Afin de permettre une reconnaissance de l'utilisateur, les signaux électrocardiographiques doivent être obtenus dans les mêmes conditions que le signal de référence correspondant au profil utilisateur sélectionné.

Selon un aspect de l'invention, le procédé de commande comprend une étape de déclaration dans laquelle un profil utilisateur est créé et/ou modifié puis enregistré dans la base de données en association avec un identifiant unique, l'étape de déclaration étant réalisée préalablement à l'étape d'élaboration d'une information de commande.

Pour définir une information de commande en fonction de l'identifiant unique sélectionné, il est nécessaire au préalable d'associer un profil utilisateur à cet identifiant unique.

Selon un aspect de l'invention, le procédé de commande comprend une étape préalable d'initialisation dans laquelle un signal électrocardiographique de référence est mesuré entre la première et la seconde électrode respectivement positionnées sur l'emplacement prédéterminé de la partie droite et l'emplacement prédéterminé de la partie gauche du corps de l'utilisateur, ledit signal électrocardiographique de référence mesuré étant associé à un identifiant unique, cette association étant enregistrée dans la base de données.

Cette disposition permet de mémoriser la signature unique du signal électrocardiographique d'un utilisateur.

La présente invention concerne également un procédé de mesure d'impédancemétrie d'un utilisateur comprenant les étapes du procédé de commande tel que décrit ci-avant et une étape additionnelle de mesure d'une impédance entre la première électrode et la seconde électrode suite à l'émission d'un signal d'excitation par le dispositif électronique de pesée.

Selon un aspect de l'invention, la mesure d'impédance est réalisée par quatre électrodes : la première électrode et la seconde électrode pour la mesure d'une différence de potentiel et deux électrodes additionnelles pour l'émission du signal d'excitation.

Cette disposition permet donc de mutualiser les première et seconde électrodes de mesure de différence de potentiel entre la mesure d'impédance et la mesure du signal électrocardiographique.

Ainsi, ce procédé est adapté pour être appliqué à un pèse personne impédancemétrique. Selon un exemple d'utilisation, le pèse personne est éteint. L'utilisateur monte sur le pèse personne afin d'obtenir son poids ainsi qu'une analyse physiologique par mesure d'impédance.

Le pèse personne détecte la présence de l'utilisateur et s'allume. Le pèse personne effectue, dans un ordre non contraint, la mesure de l'électrocardiogramme de l'utilisateur, de son poids ainsi qu'une mesure bioimpédancemétrique.

Le signal de l'électrocardiogramme est, dans un premier temps, mis en forme et converti dans le domaine numérique. Ce résultat est ensuite traité et comparé avec la base de données locale des utilisateurs afin de l'identifier.

Le résultat est affiché sur l'afficheur du pèse personne avec les différentes mesures réalisées qui peuvent être également enregistrées en lien avec le profil utilisateur en question.

La présente invention concerne également un système électronique comprenant un dispositif électronique de pesée, le système électronique étant agencé pour mettre en oeuvre les étapes d'un procédé de commande tel que décrit ci-avant ou d'un procédé de mesure d'impédancemétrie tel que décrit ci-avant et comprenant la première électrode, la seconde électrode et un étage de conditionnement pourvu d'un convertisseur analogique numérique apte à convertir le signal électrocardiographique acquis de type analogique en signal de type numérique.

Selon un aspect de l'invention, le système électronique comprend la base de données. Selon un autre aspect de l'invention, les signaux électrocardiographiques de référence sont de type numérique et de préférence sont enregistrés dans la base de données. Selon un aspect de l'invention, les profils utilisateur sont enregistrés dans la base de données.

Selon un aspect de l'invention, l'étage de conditionnement comprend un amplificateur d'instrumentation, en particulier à fort gain et/ou ayant un taux de réjection de mode commun élevé engendrant un signal différentiel à partir du signal électrocardiographique acquis.

Le signal différentiel est ensuite converti en signal numérique à l'aide du convertisseur analogique numérique, qui est de préférence de type différentiel.

Selon un aspect de l'invention, le système électronique comprend un étage décisionnel configuré pour la sélection, à partir du signal électrocardiographique acquis converti, d'un identifiant unique particulier parmi la pluralité d'identifiants uniques enregistrés dans la base de données, chaque identifiant unique de la base de données étant associé d'une part à un signal électrocardiographique de référence enregistré dans la base de données et d'autre part à un profil utilisateur enregistré dans la base de données, ledit profil utilisateur comprenant des préférences d'utilisation du dispositif électronique de pesée.

Selon un aspect de l'invention, l'étage décisionnel est pourvu d'un calculateur comprenant une unité de traitement de l'information pour la comparaison du signal électrocardiographique acquis converti avec les signaux électrocardiographiques de référence.

De préférence l'unité de traitement de l'information est agencée pour appliquer un algorithme d'apprentissage automatique. En particulier, l'algorithme d'apprentissage automatique est enregistré en mémoire de l'unité de traitement de l'information. Ce type d'algorithme est également connu sous le terme anglais « machine learning ». Selon un aspect de l'invention, le calculateur comprend un processeur.

De préférence, l'application de l'algorithme d'apprentissage automatique correspond à une modélisation statistique de manière à reconnaître un signal électrocardiographique acquis parmi une pluralité de signaux électrocardiographiques acquis.

En particulier la modélisation statistique peut être appliquée par un arbre de décisions et/ou un réseau de neurones de l'unité de traitement de l'information.

Selon un aspect de l'invention, le système électronique comprend en outre un serveur distant, l'étage décisionnel étant compris dans le serveur distant.

Cette disposition permet de limiter la puissance de calcul du dispositif électronique de pesée puisque les calculs sont déportés vers le serveur distant.

Selon un aspect de l'invention, la base de données est comprise dans le serveur distant. Cette disposition permet d'identifier un utilisateur avec tout dispositif électronique de pesée agencé pour échanger des informations avec le serveur distant.

Selon un aspect de l'invention, la première électrode et la seconde électrode sont ménagées dans des zones dédiées du dispositif électronique de pesée.

Selon un aspect de l'invention, les zones dédiées correspondent à des boutons de commande du dispositif électronique de pesée.

Selon un aspect de l'invention, les boutons de commande sont aptes à lancer une fonction du dispositif électronique de pesée. Ainsi certaines tâches peuvent être automatisées comme l'affichage d'un suivi de poids par le dispositif électronique de pesée.

Selon un aspect de l'invention, le dispositif électronique de pesée comprend un ruban équipé d'un capteur de mesure bioimpédancemétrique apte à se positionner autour de l'utilisateur au niveau de sa taille, le ruban comprenant la première électrode et la seconde électrode.

Les différents aspects définis ci-dessus non incompatibles peuvent être combinés.

### Brève description des figures

L'invention sera mieux comprise à l'aide de la description détaillée qui est exposée ci-dessous en regard du dessin annexé dans lequel :
- la figure 1 est une vue schématique d'un système de commande selon une première variante,
- la figure 2 est une vue schématique d'un système de commande selon une deuxième variante,
- la figure 3 est un schéma des étapes d'un procédé de commande d'un dispositif électronique de pesée.

### Description en référence aux figures

Dans la description détaillée qui va suivre des figures définies ci-dessus, les mêmes éléments ou les éléments remplissant des fonctions identiques pourront conserver les mêmes références de manière à simplifier la compréhension de l'invention.

### Système électronique

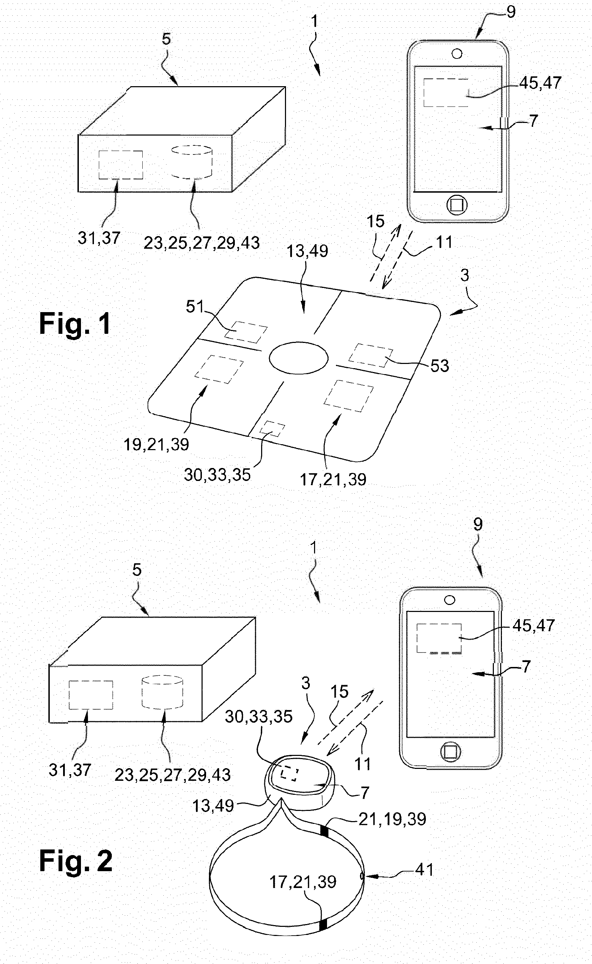

Comme illustré aux figures 1 et 2, un système électronique 1 comprend un dispositif électronique de pesée 3. Le système électronique 1 comprend également un serveur 5 distant et une interface utilisateur 7.

L'interface utilisateur 7 est ici partiellement déportée sur un terminal de communication 9 apte à échanger des informations à la fois avec le serveur 5 et le dispositif électronique de pesée 3. L'interface utilisateur 7 est également partiellement intégrée dans le dispositif électronique de pesée 3.

Ainsi le dispositif électronique de pesée 3 est agencé pour échanger des informations d'une part avec l'interface utilisateur 7 et d'autre part avec le serveur 5. La communication est réalisée sans fil par exemple selon un ou des protocoles de communication par ondes radio entre le terminal de communication 9, le serveur 5 et le dispositif électronique de pesée 3.

Le dispositif électronique de pesée 3 est apte à réaliser une fonction suite à la réception d'une information de commande 11. Selon une première variante de la figure 1 il s'agit de la mesure d'un poids, selon une deuxième variante de la figure 2 il s'agit de la mesure d'une longueur.

Le dispositif électronique de pesée 3 des figures 1 et 2 comprend également une unité de mesure 13 agencée pour mesurer une grandeur physique. A la figure 1 il s'agit d'un capteur de poids et à la figure 2 d'un capteur apte à mesurer une longueur à partir d'un ruban.

L'unité de mesure 13 est également agencée pour élaborer une information de mesure 15 relative à la mesure réalisée.

Le système électronique 1 comprend également une première électrode 17 et une deuxième électrode 19, lesdites électrodes 17, 19 étant configurées pour acquérir un signal électrocardiographique 21.

Le serveur 5 distant comprend une base de données 23 comprenant une pluralité d'identifiants uniques 25 enregistrés, chaque identifiant unique 25 étant associé à un signal électrocardiographique de référence 27 et un profil utilisateur 29 comprenant des préférences d'utilisation du dispositif électronique de pesée 3.

Les signaux électrocardiographiques de références 27 et les profils utilisateurs 29 sont également enregistrés dans la base de données 23. Les signaux électrocardiographiques de références 27 sont sous forme de données numériques.

Le dispositif électronique de pesée 3 comprend en outre un étage de conditionnement 30 pourvu d'un convertisseur analogique numérique 33 permettant de convertir le signal électrocardiographique acquis 21 par les électrodes 17, 19 en un signal numérique apte à être comparé avec un des signaux électrocardiographique de référence 27.

L'étage de conditionnement 30 comprend un amplificateur d'instrumentation 35, en particulier à fort gain et/ou ayant un taux de réjection de mode commun élevé engendrant un signal différentiel à partir du signal électrocardiographique acquis 21.

Le signal différentiel est ensuite converti en signal numérique à l'aide du convertisseur analogique numérique 33, qui est de préférence de type différentiel.

Le serveur 5 comprend un étage décisionnel 31 pourvu d'un calculateur 37 comprenant une unité de traitement de l'information pour la comparaison du signal électrocardiographique acquis 21 converti avec les signaux électrocardiographiques de référence 27.

L'unité de traitement de l'information est également agencée pour comprendre en mémoire et appliquer un algorithme d'apprentissage automatique. Ce type d'algorithme est également connu sous le terme anglais « machine learning ».

### Procédé de commande

Comme illustré à la figure 4, un procédé de commande du dispositif électronique de pesée 3 comprend les étapes détaillées ci-après.

Le procédé de commande comprend une étape E1 de mise en contact de la première électrode 17 avec un emplacement prédéterminé d'une partie droite du corps d'un utilisateur, et de la seconde électrode 19 avec un emplacement prédéterminé d'une partie gauche du corps de l'utilisateur.

La partie droite et la partie gauche du corps sont entendues comme étant respectivement du côté droit et du côté gauche du plan parallèle au plan sagittal du corps de l'utilisateur et traversant le coeur.

La première électrode 17 et la seconde électrode 19 sont ménagées dans des zones dédiées 39 du dispositif électronique de pesée 3.

A la figure 1, les zones dédiées 39 correspondent aux zones de la balance sur lesquelles l'utilisateur pose respectivement son talon gauche et son talon droit.

A la figure 2, le ruban présente un repère 41 devant être positionné dans le plan sagittal et indiquant le côté gauche et le côté droit pour le placement des électrodes 17, 19.

Ainsi les emplacements prédéterminés sont toujours les mêmes pour tous les utilisateurs.

Le procédé de commande comprend une étape E2 d'acquisition d'un signal électrocardiographique 21 entre la première électrode 17 et la seconde électrode 19.

Le procédé de commande comprend ensuite une étape E3 de sélection, à partir du signal électrocardiographique acquis 21, d'un identifiant unique 43 particulier parmi une pluralité d'identifiants uniques 43 enregistrés dans une base de données 23.

Chaque identifiant unique 43 de la base de données 23 est associé d'une part à un signal électrocardiographique de référence 27 enregistré dans la base de données 23 et d'autre part à un profil utilisateur 29 enregistré dans la base de données 23, ledit profil utilisateur 29 comprenant des préférences d'utilisation du dispositif électronique de pesée 3.

La sélection de l'identifiant unique 43 particulier est établie selon une comparaison du signal électrocardiographique acquis 21 avec les signaux électrocardiographiques de référence 27 de la base de données 23.

L'utilisateur est ainsi reconnu par la signature unique de son signal électrocardiographique 21.

Le procédé comprend ensuite une étape E4 d'élaboration d'une information de commande 11, l'information de commande 11 étant élaborée en fonction du profil utilisateur 29 associé à l'identifiant unique 43 sélectionné.

L'information de commande 11 peut également correspondre à une commande de mise à disposition par le dispositif électronique de pesée 3 d'un élément 45 d'interface utilisateur 7 pourvu d'une sélection d'au moins une commande de fonctionnement 47 parmi une pluralité de commandes de fonctionnement 47 existantes du dispositif électronique de pesée 3.

Par exemple, à la figure 1, l'information de commande 11 correspond à une commande d'enregistrement d'une information de mesure 49 provenant de l'unité de mesure 13 du dispositif électronique de pesée 3 : il s'agit de la mesure du poids de l'utilisateur et de l'enregistrement du poids en lien avec l'utilisateur identifié.

A la figure 2, il en est de même : l'utilisateur est identifié et la ou les longueurs ensuite mesurées par le ruban sont associés en mémoire de la balance à l'utilisateur en question.

Les étapes E1 à E4 peuvent être répétées, l'emplacement prédéterminé de la partie droite et respectivement l'emplacement prédéterminé de la partie gauche de l'utilisateur étant identiques à chaque étape d'acquisition d'un signal électrocardiographique 21.

Cette disposition est particulièrement intéressante lorsque plusieurs utilisateurs se servent à tour de rôle du dispositif électronique de pesée 3.

Dans le cas des figures 1 et 2, le principe est le même : l'utilisateur est reconnu en même temps qu'il effectue sa mesure, ce qui permet de changer d'utilisateur rapidement et sans confusion dans l'attribution des valeurs mesurées à un profil utilisateur en particulier.

Il est nécessaire de créer préalablement un profil utilisateur 29 et également de définir les préférences utilisateurs liées, ces préférences pouvant être modifiées ultérieurement.

Pour ce faire le procédé de commande comprend une étape E0 de déclaration dans laquelle un profil utilisateur 29 est créé et/ou modifié puis enregistré dans la base de données 23 en association avec un identifiant unique 43.

Le procédé comprend en outre une étape E00 préalable d'initialisation dans laquelle un signal électrocardiographique de référence 27 est mesuré entre la première électrode 17 et la seconde électrode 19 respectivement positionnées sur l'emplacement prédéterminé de la partie droite et l'emplacement prédéterminé de la partie gauche du corps de l'utilisateur, ledit signal électrocardiographique de référence 27 mesuré étant associé à un identifiant unique 43, cette association étant enregistrée dans la base de données 23.

Cette manipulation peut être réalisée par interaction avec l'interface utilisateur 7, l'affichage de l'interface utilisateur 7 pouvant guider l'utilisateur dans la configuration de son profil 29.

Ainsi une fois le profil utilisateur 29 défini, l'identification de l'utilisateur auprès du dispositif électronique de pesée 3 est très rapide. Il n'y a aussi aucun risque d'être connecté avec un mauvais profil utilisateur 29, par exemple sous le profil 29 de l'utilisateur précédent.

### Procédé de mesure d'impédancemétrie

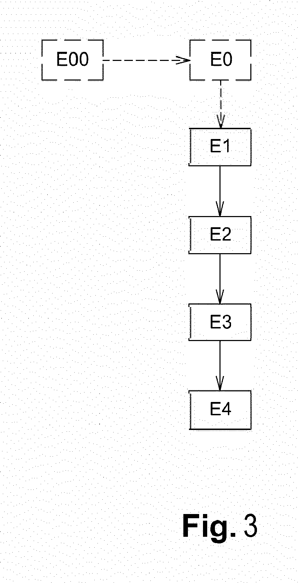

La première électrode 17 et la deuxième électrode 19 sont des électrodes de mesure. Elles peuvent également être utilisées selon la variante de la figure 2 ou de la figure 3 pour réaliser des mesures d'impédancemétrie.

Le procédé de mesure d'impédancemétrie d'un utilisateur comprend les étapes du procédé de commande tel que décrit ci-avant et une étape additionnelle de mesure d'une impédance entre la première électrode 17 et la seconde électrode 19 suite à l'émission d'un signal d'excitation par le dispositif électronique de pesée 3.

Ainsi le dispositif électronique de pesée 3 comprend une troisième électrode 51 et une quatrième électrode 53 d'émission d'un signal dont le retour est enregistré par la première électrode 17 et la deuxième électrode 19 comme illustré à la figure 2.

Cette disposition est particulièrement intéressante dans le cas d'une balance ou d'un ruban mesureur puisque la première électrode 17 et la deuxième électrode 19 sont utilisées à la fois pour identifier l'utilisateur et pour réaliser une mesure d'impédance.

Comme il va de soi, l'invention ne se limite pas à la seule forme d'exécution décrite ci-dessus à titre d'exemple, elle en embrasse au contraire toutes les variantes de réalisation.

## Revendications

1. Procédé de commande d'un dispositif électronique de pesée (3) comprenant au moins une première électrode (17) et une seconde électrode (19), le procédé comprenant :
- (E1) une étape de mise en contact de la première électrode (17) avec un emplacement prédéterminé d'une partie droite du corps d'un utilisateur, et de la seconde électrode avec un emplacement prédéterminé d'une partie gauche du corps de l'utilisateur, la partie droite et la partie gauche du corps étant entendues comme étant respectivement du côté droit et du côté gauche du plan parallèle au plan sagittal du corps de l'utilisateur et traversant le coeur ;
- (E2) une étape d'acquisition d'un signal électrocardiographique (21) entre la première et la seconde électrode (17, 19) ;
- (E3) une étape de sélection, à partir du signal électrocardiographique acquis (21), d'un identifiant unique (43) particulier parmi une pluralité d'identifiants uniques (43) enregistrés dans une base de données (23), chaque identifiant unique (43) de la base de données (23) étant associé d'une part à un signal électrocardiographique de référence (27) enregistré dans la base de données (23) et d'autre part à un profil utilisateur (29) enregistré dans la base de données (23), ledit profil utilisateur (29) comprenant des préférences d'utilisation du dispositif électronique de pesée (3) ;
- (E4) une étape d'élaboration d'une information de commande (11), l'information de commande (11) étant élaborée en fonction du profil utilisateur (29) associé à l'identifiant unique (43) sélectionné.

2. Procédé de commande selon la revendication 1, dans lequel l'étape (E3) de sélection de l'identifiant unique (43) particulier est établie selon une comparaison du signal électrocardiographique acquis (21) avec les signaux électrocardiographiques de référence (27) de la base de données (23).

3. Procédé de commande selon l'une des revendications 1 ou 2, dans lequel l'information de commande (11) correspond à une commande d'enregistrement d'une information de mesure (49) provenant d'une unité de mesure (13) du dispositif électronique de pesée (3).

4. Procédé de commande selon l'une des revendications 1 à 3, dans lequel l'information de commande (11) correspond à une commande de mise à disposition par le dispositif électronique de pesée (3) d'un élément (45) d'interface utilisateur (7) pourvu d'une sélection d'au moins une commande de fonctionnement (47) parmi une pluralité de commandes de fonctionnement (47) existantes du dispositif électronique de pesée (3).

5. Procédé de commande selon l'une des revendications 1 à 4, dans lequel les étapes de la revendication 1 sont répétées, l'emplacement prédéterminé de la partie droite et respectivement l'emplacement prédéterminé de la partie gauche de l'utilisateur étant identiques à chaque étape (E2) d'acquisition d'un signal électrocardiographique (21).

6. Procédé de commande selon l'une des revendications 1 à 5, comprenant une étape de déclaration (E0) dans laquelle un profil utilisateur (29) est créé et/ou modifié puis enregistré dans la base de données (23) en association avec un identifiant unique (43), l'étape de déclaration (E0) étant réalisée préalablement à l'étape (E4) d'élaboration d'une information de commande (11).

7. Procédé de commande selon l'une des revendications 1 à 6, comprenant une étape préalable d'initialisation (E00) dans laquelle un signal électrocardiographique de référence (27) est mesuré entre la première et la seconde électrode (17, 19) respectivement positionnées sur l'emplacement prédéterminé de la partie droite et l'emplacement prédéterminé de la partie gauche du corps de l'utilisateur, ledit signal électrocardiographique de référence (27) mesuré étant associé à un identifiant unique (43), cette association étant enregistrée dans la base de données (23).

8. Procédé de mesure d'impédancemétrie d'un utilisateur comprenant les étapes du procédé de commande selon l'une des revendications 1 à 7 et une étape additionnelle de mesure d'une impédance entre la première électrode (17) et la seconde électrode (19) suite à l'émission d'un signal d'excitation par le dispositif électronique de pesée (3).

9. Système électronique (1) comprenant un dispositif électronique de pesée (3), le système électronique (1) étant agencé pour mettre en oeuvre les étapes d'un procédé selon l'une des revendications 1 à 8 et comprenant la première électrode (17), la seconde électrode (19) et un étage de conditionnement (30) pourvu d'un convertisseur analogique numérique (33) apte à convertir le signal électrocardiographique (21) acquis de type analogique en signal de type numérique.

10. Système électronique (1) selon la revendication 9, comprenant un étage décisionnel (31) configuré pour la sélection, à partir du signal électrocardiographique acquis (21) converti, d'un identifiant unique (43) particulier parmi la pluralité d'identifiants uniques (43) enregistrés dans la base de données (23), chaque identifiant unique (43) de la base de données (23) étant associé d'une part à un signal électrocardiographique de référence (27) enregistré dans la base de données (23) et d'autre part à un profil utilisateur (29) enregistré dans la base de données (23), ledit profil utilisateur (29) comprenant des préférences d'utilisation du dispositif électronique de pesée (3).

11. Système électronique (1) selon la revendication 10, dans lequel l'étage décisionnel (31) est pourvu d'un calculateur comprenant une unité de traitement de l'information pour la comparaison du signal électrocardiographique acquis (21) converti avec les signaux électrocardiographiques de référence (27).

12. Système électronique (1) selon l'une des revendications 9 à 11, dans lequel la première électrode (17) et la seconde électrode (19) sont ménagées dans des zones dédiées (39) du dispositif électronique de pesée (3).

13. Système électronique (1) selon l'une des revendications 9 à 12, dans lequel le dispositif électronique de pesée (3) comprend un ruban équipé d'un capteur de mesure bioimpédancemétrique apte à se positionner autour de l'utilisateur au niveau de sa taille, le ruban comprenant la première électrode (17) et la seconde électrode (19).
